# EUROPEAN PATENT SPECIFICATION

(11) **EP 1 898 821 B1**
(45) Date of publication and mention of the grant of the patent: **11.12.2013**
(21) Application number: 06750667.5
(22) Date of filing: 19.04.2006
(51) Int. Cl.: A61B 18/02, A61B 18/18

(54) **COILED INJECTION TUBE**
AUFGEROLLTER INJEKTIONSSCHLAUCH
TUBE D'INJECTION EN HELICE

(30) Priority: 13.05.2005 US 129044
(43) Date of publication of application: 19.03.2008
(73) Proprietor: MEDTRONIC CRYOCATH LP, Toronto, ON M5L 1B9 (CA)
(72) Inventor: MARTIN, Robert, Lutz, Florida 33549 (US); CAPUANO, Leonilda, Pointe-claire, Quebec, H9S 5C4 (CA); MIHALIK, Teresa, Ann, Montreal, Quebec H4C 3P4 (CA)
(74) Representative: Casey, Lindsay Joseph
(86) International application number: PCT/US2006/014677
(87) International publication number: WO 2006/124184

(56) References cited:
- WO-A-2004/019798
- US-A1- 2001 037 081
- US-A1- 2002 049 436
- US-A1- 2003 088 240
- US-B1- 6 716 236

## Description

### FIELD OF THE INVENTION

The present invention relates to a medical device, and more particularly to a coolant injection tube for a thermal treatment medical device.

### BACKGROUND OF THE INVENTION

Medical devices are known for thermally treating tissue on the exterior and the interior of the body. One category of such devices is the minimally-invasive, catheter-based device that is introduced into the vasculature. One feature of these devices is means by which the device is positioned at the treatment site. For example, some devices are actively steered through the vasculature using a steering or deflection mechanism, such as a pull-wire; whereas other devices are introduced over a wire that has already been guided to a selected location, wherein the wire acts as a guide that leads the device to the treatment site. Although a device can be configured so that the guiding wire is substantially external to the device, most known devices include a central longitudinal lumen that receives the wire.

Another feature of the minimally-invasive, catheter-based, thermal-treatment device is the thermal treatment mechanism. One category of devices thermally treats tissue by cooling it, wherein cooling is effected by injecting coolant into a portion of the device, such as a distal device portion that has advantageous thermal transfer properties, and placing the distal device portion near or in contact with the tissue. The distal end portion can have a fixed diameter that is substantially the same as the diameter of the remainder of the catheter or it can have a variable diameter, such as is provided by a balloon. However, regardless of whether the whether the distal end portion is of fixed or variable diameter, the overall size of the device and the injection tube are limited by the dimensions of the vasculature. Typical devices are 5mm to 7mm in diameter. Given the small device size, it has proven challenging to cool or freeze warm bodily tissue to a temperature near or well below freezing. Accordingly, it is important to maximize the cooling potential of the coolant by delivering or injecting it at a particular location within the device.

In order to cool other than a small spot, devices as depicted in United States Patent No. 6,235,019 provide multiple coolant injection tubes. Alternately, as shown in United States Patent No. 5,899,898, a single injection tube can be provided with openings along its length. Although such coolant injection structures can be very desirable for created an elongated cooling zone, they are less suitable for balloon devices or over-the-wire devices. With respect to an over-the-wire device, it will be noted that a relatively large central passage for the wire actually blocks or isolates the injection lying at one side of the passage from the opposite side of the passage, thereby insulating the masked side of the device and creating uneven cooling.

Although not directed to issues related to an over-the-wire device, United States Patent No, 6,551,274 illustrates a loosely coiled injection tube. However, as with the linear injection tubes having a series oflongitudinal ports, at regular intervals along the device, the central structure masks the injection tube.

In view of the preceding, it is believed that an improved injection tube would be desirable for use with over-the-wire devices or other devices that have structures other than an injection tube in the cooling chamber of the device.

International Patent specification no W02004/019798 pertains to devices for causing cold-induced necrosis or apoptosis. The devices include a cryotherapy apparatus including a core member; a cryoplasty tube coupled to the core member, the cryoplasty tube having a proximal end and a distal end; wherein the distal end includes a coil disposed about at least a portion of the tubular sheath, the coil including at least one opening; an outer tube disposed over at least a portion of the cryoplasty tube; and a cooling member disposed over the coil and coupled to the outer tube. A method of causing cold-induced necrosis is also disclosed.

United States Patent specification no US-A-2003/088240 discloses methods and apparatus for cryotherapy. A hollow guidewire is disposed within a catheter having helical loops contacting tissue. A coolant delivery tube disposed within can have a coolant delivered from a proximal end into the guidewire lumen. The coolant flows back proximally through the guidewire while cooling the guidewire surface and cooling or cryogenically ablating the contacting tissue. To minimize guidewire exposure to surrounding fluids or tissue, insulative barriers can be attached to the guidewire. A coolant delivery tube and return lumen can be integrated from a single extrusion in various configurations.

Document US 2001/037081 discloses a device according to the preamble of claim 1.

### SUMMARY OF THE INVENTION

According to an aspect of the present invention, there is provided a medical device as specified in claim 1.

The present invention provide an improved injection tube for use with over-the-wire devices or devices that have structures other than an injection tube in the cooling chamber of the device. The medical device, according to the invention, includes a steering element and a fluid injection tube, wherein a portion of the fluid injection tube is wound around the steering element. The steering element can include a passage for a guide wire.

More particularly, the medical device includes a catheter having a proximal end and a distal end, the catheter defining a lumen; a passage for a guide wire disposed within the lumen so as to be coaxial with the lumen and having an open proximal end that is substantially coterminous with the proximal end of the catheter and an open distal end that is substantially coterminous with the distal end of the catheter; a fluid injection tube, wherein a portion of the fluid injection tube is wound around the passage for the guide wire; and a plurality of radially spaced injection ports in the portion of the fluid injection tube that is wound around the passage for the guide wire.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 is side section view showing the interior of a catheter based medical device in accordance with the invention; and
FIG. 2 illustrates an alternate embodiment of the catheter based medical device shown in FIG. 1.

### DETAILED DESCRIPTION OF THE INVENTION

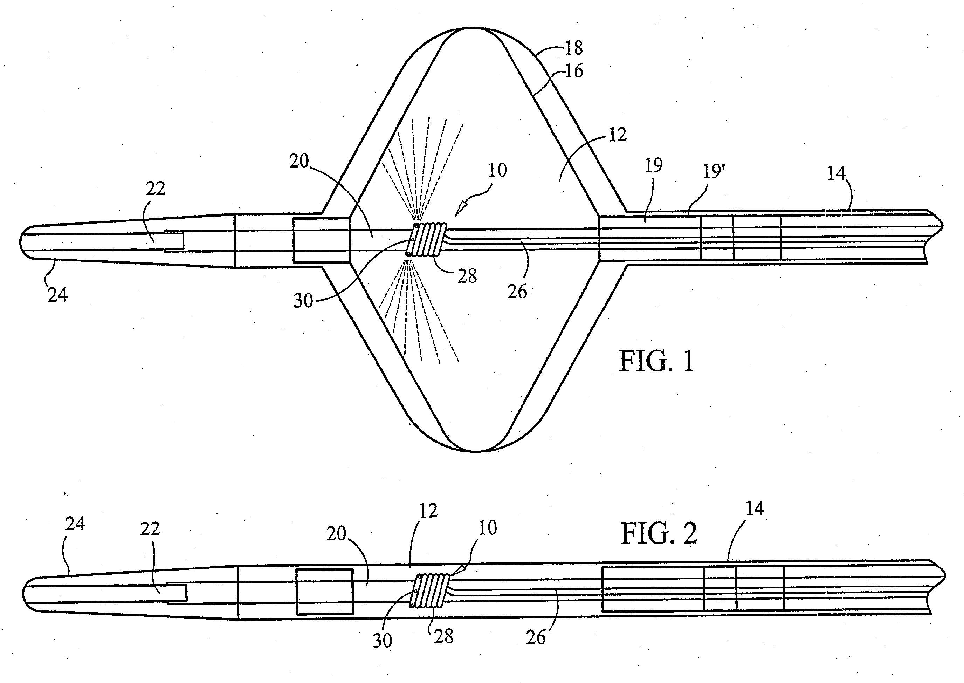

Referring to FIGS. 1 and 2, a medical device in accordance with the invention is illustrated. The device includes a fluid injection tube 10 disposed within a lumen 12, space or void defined by a portion of a catheter 14. As shown in FIG. 1, the lumen 12 is defined by a first balloon 16 encapsulated within a second balloon 18. The second balloon 18 contains leaks in the first balloon 16 should they occur and the gap shown between the balloons for the purpose of illustration does not exist when the inner balloon is inflated. Low pressure or vacuum return lumens 19 and 19' are in fluid communication with the interior of the first and second balloons, respectively.

However, referring to FIG. 2, the lumen 12 can also be a substantially uniform diameter passage within a wall portion of the catheter 14, one lumen of a multi-lumen configuration, or central lumen within a catheter that is coaxial with the longitudinal axis of the catheter.

At least a portion of the fluid injection tube 10 is wound around a structure 20 that passes through or is contained within the lumen 12 such as another tube, a wire, a shim, or a spring. In the illustration of FIGS. 1 and 2, the structure 20 is part of a catheter steering element, namely, a tube that defines a passage 22 or lumen for a guide wire (not shown). As shown, the passage 22 has an open proximal end that is substantially coterminous with the proximal end of the catheter and an open distal end that is substantially coterminous with the distal end of the catheter. The guide wire is suitable for placement into the vasculature of a patient and the passage 22 slides over the wire (i.e., the wire goes through the passage), for guiding the distal portion of the catheter to a desired location using techniques known in the art. The distal end of the catheter can include a soft tip element 24.

Continuing to refer to FIGS. 1 and 2, the fluid injection tube 10 includes a longitudinal portion 26 in fluid communication with a helically wound portion 28, wherein the helically wound portion 28 is wrapped around a portion of the passage 22 and the longitudinal portion 26 is disposed adjacent and exterior to the passage 22. In an exemplary embodiment, the helically wound portion 28 includes at two or more windings (in the illustrated embodiment there are six windings) that span an axial distance along the passage 22 about 2.54 mm (about 0.1 inches). Although the fluid injection tube 10 can be tacked or firmly bonded to the exterior of the passage 22, it can alternatively be secured to the passage 22 only by the encirclement thereof by the helically wound portion 28 so that the fluid injection tube and the passage can be axially movable with respect to each other.

The fluid injection tube 10 can be apertured or plugged at its distal end, and/or it can include multiple radially-spaced injection ports 30 helically wound portion 28. The radially spaced injection ports 30 can be equally spaced apart; and, in an exemplary embodiment, four injection ports 30 are spaced 90 degrees apart on the distal most winding. As to materials, the longitudinal portion 26 can be made of polyimide and helically wound portion 28 can be made of stainless steel. When coolant in liquid, gas, or mixed phase state exits the ports 30 (as shown by a stylized spray pattern), the coolant expands and/or fills the lumen or space 12 and then is evacuated through the return lumen 19.

In an exemplary embodiment, the longitudinal portion 26 includes 0.32 mm (0.0126") polyimide tubing and helically wound portion 28 is a stainless steel coil having a 0.56 mm (0.022") outer diameter and a 0.36 mm (0.014) inner diameter. There are four 0.63 mm (0.0025") laser drilled ports in the helically wound portion that are equally spaced.

It will be appreciated by persons skilled in the art that the present invention is not limited to what has been particularly shown and described herein above. In addition, unless mention was made above to the cantrary, it should be noted that all of the accompanying drawings are not to scale. A variety of modifications and variations are possible in light of the above teachings without departing from the scope of the invention, which is limited only by the following claims.

## Claims

1. A medical device for thermally affecting tissue comprising:
a steering element (20);
a fluid injection tube (10), having a helically coiled portion (28) wound around the steering element (20), wherein the helically wound portion (28) has a plurality of outwardly-facing, radially spaced injection ports (30), wherein at least a portion of the fluid injection tube (10) is firmly bonded to the steering element (20), such that movement of the steering element (20) causes movement of the fluid injection tube (10) **characterised in that** the medical device further comprises a catheter (14) having a proximal end, a distal end, and a lumen (12), wherein the steering element (20) is disposed within the lumen (12) of the catheter (14).

2. The medical device of claim 1, wherein the steering element (20) includes a passage (22) for a guide wire.

3. The medical device of claim 1, wherein the steering element (20) is coaxial with the longitudinal axis of the lumen (12).

4. The medical device of claim 3, wherein the steering element (20) has an open proximal end that is substantially coterminous with the proximal end of the catheter (14) and an open distal end that is substantially coterminous with the distal end of the catheter (14).

5. The medical device of claim 1, wherein the fluid injection tube (10) includes a longitudinal portion (26) in fluid communication with the helically wound portion (28) and the longitudinal portion (26) is disposed adjacent to an exterior of the steering element (20).

6. The medical device of claim 1, wherein the plurality of radially spaced injection ports (30) is equally spaced apart.

7. The medical device of claim 6, wherein there are four injection ports (30).

8. The medical device of claim 6, wherein the longitudinal portion (26) of the fluid injection tube (10) is made of polyimide and the helically wound portion (28) is made of stainless steel.

9. The medical device of claim 1, wherein the helically wound portion (28) of the fluid injection tube (10) includes at least two windings, the radially spaced injection ports (30) are in the distal most winding and, optionally, the at least two windings span an axial distance along the passage for the guide wire of about 0.254 cm (about 0.1 inches).

## Patentansprüche

1. Medizinisches Instrument zum Wärmebehandeln von Gewebe, das Folgendes aufweist:
ein Lenkelement (20);
einen Fluideinspritzschlauch (10) mit einem spiralförmig gewundenen Teil (28), der um das Lenkelement (20) gewickelt ist, wobei der spiralförmig gewundene Teil (28) eine Anzahl nach außen weisender, radial beabstandeter Einspritzöffnungen (30) aufweist, und mindestens ein Teil des Fluideinspritzschlauchs (10) fest an das Lenkelement (20) gebunden ist, so dass Bewegung des Lenkelements (20) Bewegung des Fluideinspritzschlauchs (10) bewirkt;
**dadurch gekennzeichnet, dass** das medizinische Instrument weiter Folgendes aufweist:
einen Katheter (14) mit einem proximalen Ende, einem distalen Ende, und einem Lumen (12), wobei das Lenkelement (20) innerhalb des Lumens (12) des Katheters (14) angeordnet ist.

2. Medizinisches Instrument nach Anspruch 1, bei dem das Lenkelement (20) einen Durchgang (22) für einen Führungsdraht enthält.

3. Medizinisches Instrument nach Anspruch 1, bei dem das Lenkelement (20) koaxial mit der Längsachse des Lumens (12) ist.

4. Medizinisches Instrument nach Anspruch 3, bei dem das Lenkelement (20) ein offenes proximales Ende, das im Wesentlichen an das proximale Ende des Katheters (14) angrenzt, und ein offenes distales Ende aufweist, das im Wesentlichen an das distale Ende des Katheters (14) angrenzt.

5. Medizinisches Instrument nach Anspruch 1, bei dem der Fluideinspritzschlauch (10) einen Längsteil (26) in Fluidkommunikation mit dem spiralförmige gewundenen Teil (28) enthält, und der Längsteil (26) angrenzend an die Außenseite des Lenkelements (20) angeordnet ist.

6. Medizinisches Instrument nach Anspruch 1, bei dem die Anzahl radial beabstandeter Einspritzöffnungen (30) in gleichem Abstand angeordnet ist.

7. Medizinisches Instrument nach Anspruch 6, bei dem vier Einspritzöffnungen (30) vorhanden sind.

8. Medizinisches Instrument nach Anspruch 6, bei dem der Längsteil (26) des Fluideinspritzrohrs (10) aus Polyimid besteht und der spiralförmig gewundene Teil (28) aus Edelstahl besteht.

9. Medizinisches Instrument nach Anspruch 1, bei dem der spiralförmig gewundene Teil (28) des Fluideinspritzrohrs (10) mindestens zwei Windungen enthält, die radial bestandeten Einspritzöffnungen (30) sich in der am weitesten distal angeordneten Windung befinden, und optional die mindestens zwei Windungen einen axialen Abstand entlang des Durchgangs für den Führungsdraht von etwa 0,254 cm (etwas 0,1 Zoll) überspannen.

## Revendications

1. Dispositif médical permettant d'affecter les tissus de manière thermique comportant :
un élément de guidage (20) ;
un tube d'injection de fluide (10), ayant une partie à enroulement en hélice (28) enroulée autour de l'élément de guidage (20), dans lequel la partie à enroulement en hélice (28) a une pluralité d'orifices d'injection orientés vers l'extérieur et espacés dans le sens radial (30), dans lequel au moins une partie du tube d'injection de fluide (10) est reliée de manière ferme à l'élément de guidage (20), de sorte que le mouvement de l'élément de guidage (20) amène le mouvement du tube d'injection de fluide (10) ;
**caractérisé en ce que** le dispositif médical comporte par ailleurs
un cathéter (14) ayant une extrémité proximale, une extrémité distale, et une lumière (12), dans lequel l'élément de guidage (20) est disposé à l'intérieur de la lumière (12) du cathéter (14).

2. Dispositif médical selon la revendication 1, dans lequel l'élément de guidage (20) comprend un passage (22) pour un fil guide.

3. Dispositif médical selon la revendication 1, dans lequel l'élément de guidage (20) est coaxial par rapport à l'axe longitudinal de la lumière (12).

4. Dispositif médical selon la revendication 3, dans lequel l'élément de guidage (20) a une extrémité proximale ouverte qui est sensiblement contiguë par rapport à l'extrémité proximale du cathéter (14) et une extrémité distale ouverte qui est sensiblement contiguë par rapport à l'extrémité distale du cathéter (14).

5. Dispositif médical selon la revendication 1, dans lequel le tube d'injection de fluide (10) comprend une partie longitudinale (26) en communication fluidique avec la partie à enroulement en hélice (28) et la partie longitudinale (26) est disposée de manière adjacente par rapport à un extérieur de l'élément de guidage (20).

6. Dispositif médical selon la revendication 1, dans lequel la pluralité d'orifices d'injection espacés dans le sens radial (30) sont espacés de manière équidistante.

7. Dispositif médical selon la revendication 6, dans lequel il y a quatre orifices d'injection (30).

8. Dispositif médical selon la revendication 6, dans lequel la partie longitudinale (26) du tube d'injection de fluide (10) est réalisée en polyimide et la partie à enroulement en hélice (28) est réalisée en acier inoxydable.

9. Dispositif médical selon la revendication 1, dans lequel la partie à enroulement en hélice (28) du tube d'injection de fluide (10) comprend au moins deux enroulements, les orifices d'injection espacés dans le sens radial (30) sont dans l'enroulement le plus distal et, éventuellement, lesdits au moins deux enroulements recouvrent une distance axiale le long du passage pour le fil guide d'environ 0,254 cm (environ 0,1 pouces).
